# EUROPÄISCHE PATENTANMELDUNG

(11) **EP 1 181 923 A2**
(43) Veröffentlichungstag der Anmeldung: **27.02.2002**
(21) Anmeldenummer: 01118890.1
(22) Anmeldetag: 17.08.2001
(51) Int. Cl.: A61K 6/083

(54) **Dentalmaterialien enthaltend ein Abreissmaterial**

(30) Priorität: 21.08.2000 DE 10040780
(71) Anmelder: Ivoclar Vivadent AG, 9494 Schaan (LI)
(72) Erfinder: Hagenbuch, Konrad, 9472 Grabs (CH); Zanghellini, Gerhard, 9494 Schaan (LI)
(74) Vertreter: UEXKÜLL & STOLBERG

(57) **Zusammenfassung**

Dentalmaterial enthaltend eine polymerisierbare Komponente und eine oberflächlich auf die polymerisierbare Komponente aufgebrachte Komponente, die sich nach dem Härten der polymerisierbaren Komponente unter Bildung einer rauhen Oberfläche entfernen läßt.

## Beschreibung

Die Erfindung betrifft Dentalmaterialien auf der Basis polymerisierbarer Stoffe, die ein sogenanntes Abreißmaterial enthalten, d.h. eine Komponente, die sich nach dem Härten der polymerisierbaren Komponente unter Bildung einer rauhen Oberfläche von dem gehärteten Dentalmaterial entfernen (abreißen) läßt.

Die US 5,839,900 offenbart ein Verfahren zur Herstellung von Dentalrestaurationen, bei dem mit organischem Matrixmaterial getränkte Gewebestücke auf ein Modell aufgelegt, durch Anpressen an das Modell in einem Tiefziehverfahren geformt und anschließend gehärtet werden. Nach dem Härten wird das so erhaltene Grundgerüst durch das Aufbringen eines Deckmaterials verblendet. Zur Gewährleistung einer sicheren Haftung zwischen Deckschicht und Grundgerüst wird letzteres vor dem Verblenden aufgerauht. Hierbei ist eine Verletzung der in die Polymermatrix eingebetteten Glasfasern häufig nicht zu vermeiden und zudem besteht die Gefahr, daß zuviel Material abgetragen und somit instabile Strukturen erhalten werden.

Die DE 31 09 424 A1 offenbart ein Verfahren zur Herstellung faserverstärkter Kunststoffgegenstände, bei dem mit härtbaren Harzen imprägnierte Fasern, die ein- oder beidseitig mit einer flexiblen, dehnbaren Folie bedeckt sind, auf eine geeignete Form gelegt, durch Vakuumformung an diese angepaßt und dann gehärtet werden. Die Folien verbleiben nach der Polymerisation entweder auf dem gehärteten Kunststoff oder werden nach dem Härten entfernt. In beiden Fällen werden Gegenstände mit glatter Oberfläche erhalten.

Aufgabe der vorliegenden Erfindung ist die Bereitstellung von Dentalmaterialien, die nach dem Härten ohne zusätzliches Aufrauhen und Reinigen eine zur direkten Weiterverarbeitung geeignete Oberfläche aufweisen.

Diese Aufgabe wird durch Dentalmaterialien auf der Basis von polymerisierbaren Substanzen gelöst, die eine oberflächlich auf die polymerisierbare Komponente aufgebrachte Komponente enthalten, die sich nach dem Härten der polymerisierbaren Komponente unter Bildung einer rauhen Oberfläche entfernen (abreißen) läßt. Die oberflächlich aufgebrachte Komponente wird im folgenden auch als Abreißmaterial bezeichnet.

Als oberflächlich aufgebrachte Komponente oder Abreißmaterial eignen sich insbesondere mattenförmige Materialien, die eine strukturierte Oberfläche aufweisen. Beim Aufbringen auf die ungehärtete und damit noch formbare polymerisierbare Komponente wird die Struktur des Abreißmaterials auf die polymerisierbare Komponente übertragen und diese damit gewissermaßen geprägt. Bei Härten der polymerisierbaren Komponente wird die Oberflächenstruktur eingefroren und bleibt nach dem Entfernen des Abreißmaterials erhalten.

Als Abreißmaterial können Folien oder vorzugsweise Gewebe verwendet werden.

Unter Folien werden mattenförmige Materialien verstanden, die durch eine homogene Polymermatrix gekennzeichnet sind und beispielsweise durch Extrusion geeigneter gefüllter oder ungefüllter Polymermaterialien hergestellt werden können.

Vorzugsweise ist mindestens eine Oberfläche der Folien strukturiert, beispielsweise durch Prägen oder Perforieren. Perforierte Folien sind bevorzugt. Die Perforation erlaubt den Durchtritt von polymerisierbarem Material, das beim Abziehen der Folie zusammen mit der Folie entfernt wird, so daß eine besonders feine Aufrauhung der Oberfläche erzielt wird.

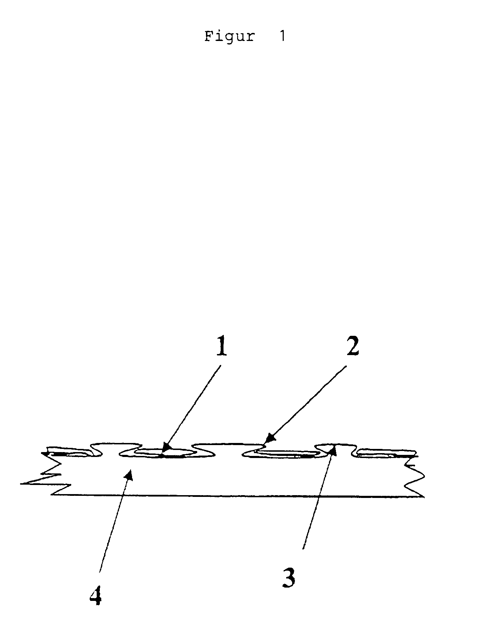

Die Funktion einer perforierten Folie ist in Figur 1 schematisch dargestellt. Figur 1 zeigt einen Längsschnitt durch ein mit einer perforierten Folie **1** bedecktes polymerisierbares Material **4**. Der Schnitt ist durch eine Reihe von Perforationen gelegt. Ein Teil des polymerisierbaren Materials ist durch die Perforationen **3** hindurchgetreten und auf der freien Seite der Folie tellerförmig verlaufen **2**. Hierdurch wird die Folie bei der Härtung oberflächlich an das polymerisierbare Materials gebunden. Die tellerförmig erweiterten Bereiche **2** des polymerisierbaren Materials **4** werden beim Abreißen der Folie zusammen mit der Folie **1** entfernt.

Die Perforationen haben vorzugsweise einen kreisförmigen Querschnitt. Eine bevorzugte perforierte Folie weist Perforationen mit einem Durchmesser von etwa 0.4 mm im Abstand von etwa 0,5 bis 5 mm auf. Die Perforationen sind beispielsweise in Form von mehreren Reihen angeordnet, wobei der Abstand der Perforationen innerhalb der Reihe vorzugsweise 0,5 bis 5 mm und der Abstand zwischen den Reihen vorzugsweise ebenfalls 0,5 bis 5 mm beträgt, wobei die einzelnen Reihen versetzt gegeneinander angeordnet sein können.

Unter Geweben werden mattenförmige Materialien verstanden, die aus Fäden oder Fasern gewebt sind. Beim Aufbringen des Gewebes auf die polymerisierbare Komponente kann Matrixmaterial in das Gewebe eindringen und die Fasern umschließen. Bei der anschließenden Härtung wird das Abreißgewebe an die Oberfläche des Werkstoffs gebunden. Beim Abziehen des Abreißgewebes wird der in das Gewebe eingedrungene Matrixfilm von der Oberfläche des Kunststoffkörpers abgerissen wobei eine rauhe und saubere Oberfläche erhalten wird, die sich zur direkten Weiterverarbeitung eignet.

Ein weiterer Vorteil von perforierten Folien und Abreißgeweben liegt darin, daß sie es überschüssigem Harz erlauben, zu entweichen. Dieses wird nach dem Härten zusammen mit dem Gewebe entfernt.

Durch die Verwendung von Abreißmaterialien wird ein zeitaufwendiges Anrauhen der Oberfläche, beispielsweise durch Anschleifen oder Sandstrahlen, vermieden. Bei Faserverbundwerkstoffen wird zudem die Gefahr einer Beschädigung der Armierungsfasern gebannt. Da das Anrauhen mit einem erheblichen Staubanfall verbunden ist, entfällt gleichzeitig auch das anschließende Reinigen der angerauhten Gegenstände.

Als Materialien für die Abreißgewebe und Abreißfolien eignen sich besonders Cellulose (auch modifizierte Typen) und thermoplastische Kunststoffe, vorzugsweise kristalline oder semikristalline thermoplastische Kunststoffe. Bevorzugte thermoplastische Materialien sind Polyolefine, insbesondere Polyethylen (PE), Polypropylen (PP) und Polyphenylenether (PPE oder PPO), amorphe und/oder kristalline Polyester, insbesondere Polyalkylenterephthalate, wie Polyethylenterephthalat (PETP oder PET) und Polybutadienterephthalat (PBT), Polyimide (PI), wie Polyetherimide (PEI) und Polyamidimide (PA), Polyphenylensulfide (PPS), Polyvinylfasern, Polyvinylchlorid (PVC), Polyvinylidenchlorid (PVDC), Copolymere von Vinylchlorid und Vinylidenchlorid oder Polyvinylidenfluorid (PVDF oder PVF2), Polyacrylnitril (PAN) Polyarylensulfidketone, Polyoxymethylen (POM), Polycarbonat (PC), Polyether (PEth), Polyetherketone (PEK), Polyetherketonketone (PEKK), Polyetheretherketone (PEEK), Polyacetale, Polyurethane (PUR), Celluloseester und Copolymere und Mischungen dieser Materialien. Besonders bevorzugt sind Polyalkylenetherterephthalate, insbesondere Polyethylenterephthalat, und Polyamide, insbesondere Nylon, vorzugsweise Nylon 4.6, Nylon 8, Nylon 6.10, Nylon 11 und andere für textile Anwendungen gebräuchliche Nylonarten, ganz besonders Nylon 6, Nylon 6.6 und Nylon 12.

Als Gewebematerialien eignen sich zudem Glasfasern, Regeneratfasern, sowie natürliche Fasern, wie Flachs und Leinen. Unter Regeneratfasern werden chemisch modifizierte Cellulosefasern verstanden. Weiterhin können Hochleistungsfasern, d.h. Fasern mit hoher Zugfestigkeit, wie beispielsweise Aramidfasern (Aromatische Polyamid-Fasern) verwendet werden. Geeignete Aramidfasern sind unter der Marke Kevlar (DuPont) kommerziell erhältlich.

Soweit es nicht bereits aus diesen Materialien besteht, weist das Abreißmaterial vorzugsweise eine Oberfläche aus Polyester, Polyethylen oder Polyamid auf, da sich diese Materialien nach der Polymerisation der polymerisierbaren Komponente gut von der gehärteten Matrix entfernen lassen.

Aufgrund ihrer guten Drapierbarkeit sind Gewebe mit Atlasbindung (Satin-Bindung) und insbesondere Körperbindung bevorzugt. Besonders bevorzugt sind Gewebe mit Atlas-1/7-Bindung und insbesondere Körper-2/2-Bindung. Derartige Gewebe können ohne Faltenbildung leicht an dreidimensionale Körper, wie beispielsweise Zahnstümpfe, angepaßt werden. Andere Bindungsarten, wie z.B. die Leinwandbindung, eignen sich besonders für ebene Flächen.

Die Abreißgewebe haben vorzugsweise ein Gewebegewicht von 20 bis 500 g/m², vorzugsweise 40 bis 150 g/m² und besonders bevorzugt etwa 100 g/m². Das Gewebegewicht wird an die Größe des zu überziehenden Teils angepaßt, je kleiner der zu überziehende Gegenstand ist, desto niedriger sollte das Gewebegewicht gewählt werden. Gewebe mit einem Gewebegewicht von 40 bis 80 g/m² ergeben eine feinere Oberfläche, während Gewebe mit einem Gewebegewicht von > 80 bis 150 g/m² eine rauhere Oberfläche ergeben. Für die Herstellung von Zahnkronen ist beispielsweise ein Gewebegewicht von 40 bis 70 g/m² besonders vorteilhaft.

Ein ganz besonders bevorzugtes Abreißgewebe ist Nylongewebe mit einem Gewebegewicht von 98 g/m² und einer Körperbindung 2/2.

Weiter bevorzugt sind Gewebe mit einem Fadendurchmesser von 4 bis 50 µm, vorzugsweise von 10 bis 30 µm, insbesondere von 15 bis 25 µm, ganz besonders bevorzugt von 18 bis 20 µm.

Das Abreißmaterial wird auf die verwendete polymerisierbare Komponente abgestimmt. Die Eigenfestigkeit des Abreißmaterials (Reißfestigkeit) sollte größer sein als die Verbundfestigkeit zwischen Abreißmaterial und polymerisierbarer Komponente. Bei der Verwendung sehr leichter Gewebe zur Gewinnung von fein strukturierten Oberflächen ist daher die Verwendung sogenannter Hochleistungsgewebe bevorzugt, um eine ausreichende Reißfestigkeit sicherzustellen. Zur Verringerung der Abreißkräfte kann die Oberfläche des Abreißmaterials mit einer Antihaftausrüstung versehen werden, vorzugsweise durch Aufbringen von Teflonpulver oder Polyethylenwachs.

Als polymerisierbare Komponente wird ein härtbares Material verwendet, das neben einer Matrix von polymerisierbaren Monomeren, Oligomeren, Polymeren und/oder Präpolymeren vorzugsweise auch einen oder mehrere Füllstoffe, besonders bevorzugt einen faserförmigen Füllstoff, insbesondere Fasermatten und/oder uniaxial ausgerichtete Faserbündel enthält. Die gleichzeitige Verwendung von partikulären und faserförmigen Füllstoffen ist ebenfalls möglich.

Als polymerisierbare Bestandteile enthält die polymerisierbare Komponente vorzugsweise ionisch und/oder radikalisch polymerisierbare mono- oder multifunktionelle Monomere, insbesondere Mono(meth)acrylate, wie Methyl-, Ethyl-, Butyl-, Benzyl-, Furfuryl- oder Phenyl(meth)acrylat, mehrfunktionelle Acrylate und Methacrylate wie zum Beispiel Bisphenol-(A)-di(meth)acrylat, Decandioldi(meth)-acrylat, Butandioldi(meth)acrylat, 1,10-Decandioldi(meth)acrylat und/oder 1,12-Dodecandioldi(meth)acrylat.

Besonders bevorzugte polymerisierbare Bestandteile sind Polycarbonat-di(meth)acrylate, insbesondere das Kondensationsprodukt aus einem Hydroxyalkylmethacrylat, vorzugsweise 2-Hydroxyethylmethacrylat, und einem Bis(chlorformiat), vorzugsweise Triethylenglykolbis(chlorformiat)). Polycarbonat-Tri- oder Tetra-(meth)-acrylate, Urethan-di-, tri-, tetra-(meth)acrylate und Mischungen davon. Monomere dieses Typs werden in der DE 36 32 868 A1 und der US 5,444,104 beschrieben.

Weitere besonders bevorzugte Monomere sind Bis-GMA (ein Additionsprodukt aus Methacrylsäure und Bisphenol-A-diglycidylether), UDMA (ein Additionsprodukt aus 2-Hydroxyethylmethacrylat und 2,2,4-Hexamethylendiisocyanat), Di-, Tri- (TEGMA) und Tetra-ethylenglykoldi(meth)acrylat, Trimethylolpropantri(meth)acrylat und Pentaerythrittetra(meth)acrylat.

Zur Initiierung der radikalischen Polymerisation enthält die polymerisierbare Komponente vorzugsweise thermische und/oder Photoinitiatoren.

Bevorzugte Initiatoren für die thermische Härtung sind Peroxide, wie beispielsweise Dibenzoylperoxid, Dilaurylperoxid, tert.-Butylperoctoat und tert.-Butylperbenzoat sowie Azobisisobutyroethylester, Benzpinakol und 2,2-Dimethylbenzpinakol.

Bevorzugte Photoinitiatoren sind Benzophenon und Benzoin sowie deren Derivate, α-Diketone und deren Derivate, wie beispielsweise 9,10-Phenanthrenchinon, Diacetyl und 4,4-Dichlorbenzil. Besonders bevorzugte Photoinitiatoren sind Champherchinon und 2,2-Methoxy-2-phenyl-acetophenon und insbesondere Kombinationen von α-Diketonen mit Aminen als Reduktionsmittel, wie zum Beispiel N-Cyanoethyl-N-methylanilin, 4-(N,N-Dimethylamino)-benzoesäureester, N,N-Dimethylaminoethylmethacrylat, N,N-Dimethylsym.-xylidin oder Triethanolamin. Darüber hinaus sind Acylphosphine, wie zum Beispiel 2,4,6-Trimethylbenzoyldiphenyl- oder Bis-(2,6-dichlorbenzoyl)-4-N-propylphenylphosphinoxid, als Photoinitiatoren geeignet.

Für die duale Aushärtung von radikalisch und kationisch polymerisierbaren Systemen eignen sich besonders Diaryliodonium- oder Triarylsulfoniumsalze, wie zum Beispiel Triphenylsulfoniumhexafluorophosphat und -hexafluoroantimonat.

Als Initiatoren für eine Polymerisation bei Raumtemperatur werden Redox-Initiatorkombinationen, wie zum Beispiel Kombinationen von Benzoyl- oder Laurylperoxid mit N,N-Dimethyl-sym.-xylidin oder N,N-Dimethyl-p-toluidin, verwendet. Weitere geeignete Initiatoren und Beschleuniger sind Vitamin C und Barbitursäure.

Als Füllstoffe sind organische und anorganische faserförmige Materialien, insbesondere Fasermatten und/oder uniaxial ausgerichtete Faserbündel bevorzugt. Bevorzugte Fasermaterialien sind Glasfasern und Polyethylenfasern (Spectra, Dynema), Polyamidinsbesondere Aramidfasern (Kevlar) und Carbonfasern.

Bevorzugte partikuläre Füllstoffe sind amorphe kugelförmige Materialien auf der Basis von Mischoxiden aus SiO₂, ZrO₂ und/ oder TiO₂ (DE 40 29 230 A1), mikrofeine Füllstoffe, wie pyrogene Kieselsäure oder Fällungskieselsäure, sphärische SiO₂-Partikel (gefällte Partikel) mit einer Korngröße von 200 bis 700 nm sowie Makro- (Partikelgröße von 5 µm bis 200 µm) oder Minifüllstoffe (Partikelgröße von 0,5 bis 5 µm), wie Quarz-, Glaskeramik- oder Glaspulver mit einer durchschnittlichen Teilchengröße von 0,5 µm bis 5 µm sowie röntgenopake Füllstoffe, wie Ytterbiumtrifluorid.

Die Mischungen können darüber hinaus weitere Additive wie Färbemittel (Pigmente und Farbstoffe), Stabilisatoren, Aromastoffe, mikrobiozide Wirkstoffe, Weichmacher und/oder UV-Absorber enthalten.

Gemäß einer besonders bevorzugten Ausführungsform handelt es sich bei der polymerisierbaren Komponente um mit einer polymerisierbaren Matrix imprägnierte Gewebematten oder Faserbündel. Geeignete Faserverbundwerkstoffe, d.h. Kombinationen von organischer Matrix, faserförmigem Füllstoff und gegebenenfalls partikulärem Füllstoff werden in der amerikanischen Patentschrift US 5,839,900 beschrieben.

Bevorzugte Faserverbundwerkstoffe zur Verwendung mit Schrumpfmaterialien enthalten 7 bis 94 Gew.-% faserförmigen Füllstoff und 6 bis 93 Gew.-% polymerisierbare Matrix. Besonders bevorzugt sind Werkstoffe die 28 bis 82 Gew.-% und insbesondere 35 bis 65 Gew.-% faserförmige Füllstoffe enthalten.

Bevorzugt ist eine polymerisierbare Matrix auf der Basis einer Mischung von Bis-GMA, Decandioldimethacrylat (DDDMA), Triethylenglycoldimethacrylat (TEGDMA) und Urethandimethacrylat (UDMA). Eine besonders bevorzugte polymerisierbare Matrix enthält 24,5 bis 38,6 Gew.- % Bis-GMA, 0,3 bis 0,5 Gew.-% Decandioldimethacrylat, 6,2 bis 9,7 Gew.-% Triethylenglycoldimethacrylat und 0,1 Gew.-% Urethandimethacrylat. Die Prozentangaben beziehen sich auf den Faserverbundwerkstoff.

Der Anteil nicht-faserförmiger Füllstoffe liegt vorzugsweise im Bereich von 0 bis 30 Gew.-%, insbesondere 2 bis 15 Gew.-% und besonders bevorzugt im Bereich von 3,5 bis 5,5 Gew.-%. Gemäß einer ganz besonders bevorzugten Ausführungsform enthält das Material 3,5 bis 5,5 Gew.-% hoch disperse Kieselsäure als zusätzlichen Füllstoff.

Initiatoren und ggf. Beschleuniger werden vorzugsweise in einer Menge von jeweils 0,01 bis 3,0 Gew-%, besonders bevorzugt in einer Menge von jeweils 0,05 bis 1,0 Gew.-% bezogen auf die Masse an Matrixmaterial verwendet. Die Gesamtmenge an Katalysatoren und Stabilisatoren beträgt vorzugsweise jeweils 0,3 bis 0,5 Gew.-% basierend auf der Gesamtmasse des Faserverbundwerkstoffs.

Größe und Abmessung der Materialien sind auf die Verwendung bei der Herstellung von Dentalrestaurationen, wie Kronen, Brücken, Inlays und Implantataufbauten abgestimmt. Die Materialien sind so geformt, daß sie eine oder mehrere natürliche oder künstliche Implantate oder Zahnstümpfe abdecken können.

Die Gewebematten sind vorzugsweise rund mit einem Durchmesser von 10 bis 30 mm, vorzugsweise 16 bis 24 mm, oder rechteckig mit einer Länge von 15 bis 150 mm und einer Breite von 10 bis 30 mm, vorzugsweise 15 bis 25 mm.

Die polymerisierbare Komponente umfaßt vorzugsweise 2 bis 10 Lagen, vorzugsweise 3 bis 8 Lagen des imprägnierten Gewebes. Das Gewebe ist vorzugsweise ein Glasgewebe und weist bevorzugt ein Gewebegewicht von 20 g/m² bis 500 g/m², besonders bevorzugt von 70 g/m² bis 150 g/m² auf. Im Fall der runden Gewebematten sind 7 bis 9 Lagen und im Fall rechteckiger Matten 2 bis 4 Lagen eines Gewebes mit einem Gewebegewicht von 70 bis 150 g/m² bevorzugt.

Die Matten sind vorzugsweise mit einem Matrixmaterial folgender Zusammensetzung imprägniert:

| | |
|---|---|
| BisGMA: | 60 bis 80 Gew.-% |
| Triethylenglykoldimethacrylat | 10 bis 20 Gew.-% |
| Hochdisperses Siliciumdioxid | 5 bis 15 Gew.-% |
| Katalysatoren | 0,3 bis 1,5 Gew.-% |
| Stabilisatoren | 0,01 bis 0,2 Gew.-% |

Diese Materialien eignen sich insbesondere zur Herstellung von Kronen (runde Form) oder Brücken (runde und rechteckige Form).

Weiter bevorzugt sind imprägnierte Faserbündel mit unidirektionaler Faseranordnung. Diese stäbchenförmigen Materialien haben vorzugsweise eine Länge von 10 bis 160 mm, besonders bevorzugt 10 bis 40 mm und einen Durchmesser von 2 bis 4 mm, vorzugsweise 2,5 bis 3,5 mm mit rechteckigem oder vorzugsweise rundem Querschnitt. Diese Materialien werden vorzugsweise in vorgehärteter Form verwendet. Die stäbchenförmigen Materialien eignen sich beispielsweise zusammen mit den oben beschriebenen runden oder rechteckigen Materialien zur Herstellung von Brücken.

Zur Herstellung von Dentalrestaurationen wird vorzugsweise Dentalmaterial eingesetzt, daß bereits ein- oder beidseitig mit Abreißgewebe versehen ist. Ein besonders bevorzugtes Dentalmaterial ist Faserverbundwerkstoff, der ein- oder zweiseitig mit Abreißgewebe beschichtet ist und direkt auf einen natürlichen Zahnstumpf oder ein Modell aufgebracht und geformt werden kann. Stäbchenförmige Materialien werden vorzugsweise mit dem Abreißmaterial umwickelt.

Kombinationen von Abreißmaterial mit perforierten und/oder nicht perforierten Trennfilmen und Saugvliesen sind möglich. Trennfilme erleichtern das Entfernen von Dentalrestaurationen aus Formen, die zur Formung der Materialien eingesetzt werden. Saugvliese können überschüssiges Harz aufnehmen, daß bei der Formung oder dem Aufbringen des Abreißgewebes aus der polymerisierbaren Komponente austritt. Saugvlies oder Trennfilm werden ggf. als zusätzliche Schicht auf das Abreißgewebe aufgebracht.

Gegenstand der Erfindung ist auch ein Verfahren zur Herstellung von Dentalrestaurationen. Hierbei wird das polymerisierbare Material, vorzugsweise ein Faserverbundwerkstoff, auf ein geeignetes Modell, wie zum Beispiel die Reproduktion eines natürlichen Zahnstumpfes, aufgebracht. Das polymerisierbare Material wird dann mit dem Abreißmaterial bedeckt und geformt, das heißt beispielsweise durch Tiefziehen an das Modell anpaßt. Danach wird das polymerisierbare Material gehärtet und anschließend das Abreißgewebe von der Oberfläche des gehärteten Materials entfernt. Hierbei wird eine rauhe Oberfläche erhalten, die sich zur unmittelbaren Weiterverarbeitung, beispielsweise das Aufbringen von Verblendmaterialien oder das Überkronen, eignet.

Das Herstellen von Dentalrestaurationen unter Verwendung von Gewebestücken, die mit einer organischen Matrix getränkt sind, ist an sich bekannt und wird beispielsweise in der US 5,839,900 beschrieben. Eine zur Durchführung des Verfahrens geeignete Tiefziehapparatur wird ebenfalls in der US 5,839,900 beschrieben. Eine bevorzugte Apparatur zur Durchführung des Tiefziehprozesses und zur Härtung des polymerisierbaren Materials wird unter der Bezeichnung Vectris VS1 Gerüstformer von der Ivoclar AG, Liechtenstein, vertrieben.

Gemäß einer bevorzugten Variante des Verfahrens wird das zur Herstellung der Dentalrestauration verwendete Modell mit Abreißmaterial bedeckt und anschließend das polymerisierbare Material und eine weitere Lage Abreißmaterial auf das Modell aufgebracht. Nach dem Härten werden Körper erhalten, die beidseitig ein Abreißmaterial aufweisen. Auf diese Weise sind beispielsweise Kronen zugänglich, die auf der Kroneninnenseite mit Abreißmaterial versehen sind, so daß diese vom Zahnarzt nicht mehr aufgerauht werden müssen, was eine erhebliche Arbeitserleichterung darstellt.

Das Abreißgewebe kann auch nach dem Formen der polymerisierbaren Komponente aufgebracht werden kann. Wesentlich ist, daß das Aufbringen des Abreißgewebes vor dem Härten der Matrix geschieht und daß beim Aufbringen ein intensiver Kontakt zwischen der polymerisierbaren Komponente und dem Abreißgewebe sichergestellt wird.

Zur Herstellung beispielsweise einer Krone wird der zu restaurierende Zahn auf an sich bekannten Weise zu einem Stumpf geschliffen. Von diesem Stumpf wird mit Abformmaterialien, wie beispielsweise Alginat, Polyether oder Silikon, eine Negativform angefertigt, die anschließend mit einem Modellmaterial, wie Gips oder Epoxyharz, ausgegossen wird. Auf diese Weise wird ein Positivmodell erhalten, das z.B. mit einer Alginatlösung (im Fall eines Gipsmodells) oder eine Wachslösung (im Fall eines Epoxymodells) isoliert wird. Auf den isolierten Modellstumpf wird dann, wie oben beschrieben, die polymerisierbare Komponente aufgebracht, geformt und gehärtet.

Nach dem Tiefzieh- und Polymerisationsvorgang werden Überschüsse des Matrixmaterials beispielsweise mit einer Hartmetallfräse entfernt. Anschließend erfolgt die Fertigstellung der Krone mit einem geeigneten Verblendwerkstoff. Hierzu wird das Abreißgewebe von dem Kronenrohling entfernt und das Verblendmaterial direkt auf die zurückbleibende rauhe Oberfläche aufgetragen. Der Auftrag erfolgt in der Regel in zwei oder mehr Schichten, wobei die einzelnen Schichten nach dem Auftrag jeweils gehärtet werden. Nach dem Aufbringen der letzten Schicht wird die Krone endgehärtet, poliert, gereinigt und vom Zahnarzt dem Patienten eingegliedert.

Zur Herstellung einer Brücke werden die beiden der Zahnlücke benachbarten Zähne vom Zahnarzt mit geeigneten Instrumenten auf an sich bekannte Weise zu Stümpfen beschliffen und dann von der Mundsituation wie oben beschrieben ein Positivmodell angefertigt. Analog zur Herstellung einer Krone werden die Stümpfe zunächst jeweils mit einer unverblendeten Krone versehen. Anschließend wird der Zwischenraum zwischen den überkronten Pfeilerzähnen mit polymerisierbarem Material ausgefüllt. Hierzu werden vorzugsweise die oben beschriebenen stäbchenförmigen Materialien eingesetzt, die leicht auf die erforderliche Länge zugeschnitten und zwischen die Pfeilerzähne gelegt werden können. Die letzte Lage des polymerisierbaren Materials wird so aufgebracht, daß es an den Enden jeweils auf den Zahnstümpfen aufliegt und vorzugsweise die gesamte okklusale Fläche abdeckt. Vor dem Formen und Härten wird das Abreißgewebe von den Kronen und dem polymerisierbaren Material entfernt und letzteres durch Tiefziehen an das Modell angepaßt und gehärtet. Nach dem Härten wird ein brückenförmiges Zwischenglied erhalten, das beispielsweise mit einer Hartmetallfräse von überschüssigem polymerisierbarem Material befreit wird.

Zur Herstellung des Zwischengliedes wird das Modell vorzugsweise mit einer Silikonabformmasse abgedeckt, wobei lediglich der Zwischengliedbereich freigelassen wird. Hierbei kann die Anfertigung eines Wachsmodells des Zwischengliedes hilfreich sein. Die Abformmasse wird so gestaltet, daß eine okklusal zugängliche Form erhalten wird. Das polymerisierbare Material wird von oben in die Form eingebracht und wie beschrieben geformt und gehärtet.

Das gehärtete Zwischenglied wird silanisiert und wieder auf das Modell aufgesetzt. Anschließend erfolgt der Aufbau des Brückengerüsts. Hierzu wird das Zwischenglied mit polymerisierbarem Material, vorzugsweise in Form von harzgetränkten, rechteckigen Gewebematten, bedeckt, dieses durch Tiefziehen geformt und dann gehärtet. Harzüberschüsse werden entfernt und das gehärtete Brückengerüst nach dem Entfernen des Abreißgewebes verblendet. Nach dem Polieren und das Reinigen kann die fertige Brücke dem Patienten vom Zahnarzt eingegliedert werden.

Das erfindungsgemäße Verfahren zeichnet sich durch eine einfache Handhabung aus und ergibt Dentalrestaurationen mit sauberer, rauher Oberfläche, die direkt weiterverarbeitet, d.h. beispielsweise verblendet werden können. Eine Zerstörung der für die Festigkeit maßgeblichen Fasern des Faserverbundwerkstoffs durch Beschleifen, Sandstrahlen und dergleichen findet nicht statt. Durch die Verwendung des Abreißmaterials wird eine gleichbleibende, definierte Oberflächenkonditionierung sichergestellt, wobei durch die Verwendung unterschiedlicher Abreißmaterialtypen eine Steuerung der Oberflächenrauhigkeit möglich ist. Die mit dem Aufrauhen von Oberflächen üblicherweise verbundene Bildung von Staub und Schmutz wird vollständig vermieden.

Ein wesentlicher Vorteil der Erfindung ist, daß eine durch ungenaues Abtragen der gehärteten Matrix durch Beschleifen oder Sandstrahlen erfolgende Schwächung oder Zerstörung der obersten Faserlagen der Restauration vermieden wird.

Gegenstand der Erfindung sind weiterhin Kits, welche die zur Durchführung des Verfahrens erforderlichen Materialien, daß heißt mindestens ein polymerisierbares Material und ein Abreißgewebe enthalten. Vorzugsweise enthalten die Kits mehrere unterschiedliche polymerisierbare Materialien, beispielsweise runde, rechteckige und/oder stäbchenförmige Materialien und vorzugsweise auch geeignete Verblendmaterialien.

## Patentansprüche

1. Dentalmaterial enthaltend eine polymerisierbare Komponente, **dadurch gekennzeichnet, daß** es weiterhin eine oberflächlich auf die polymerisierbare Komponente aufgebrachte Komponente enthält, die sich nach dem Härten der polymerisierbaren Komponente unter Bildung einer rauhen Oberfläche entfernen läßt.

2. Dentalmaterial nach Anspruch 1, **dadurch gekennzeichnet, daß** die oberflächlich aufgebrachte Komponente eine strukturierte Oberfläche aufweist.

3. Dentalmaterial nach Anspruch 2, **dadurch gekennzeichnet, daß** die oberflächlich aufgebrachte Komponente eine perforierte Folie oder ein Gewebe ist.

4. Dentalmaterial nach Ansprüche 3, **dadurch gekennzeichnet, daß** das Gewebe ein Gewebegewicht von 20 bis 500 g/m² aufweist.

5. Dentalmaterial nach Anspruch 3 oder 4, **dadurch gekennzeichnet, daß** das Gewebe eine Atlasbindung oder Körperbindung aufweist.

6. Dentalmaterial nach einem der Ansprüche 1 bis 5, **dadurch gekennzeichnet, daß** die oberflächlich aufgebrachte Komponente Cellulose, Glas oder thermoplastischen Kunststoff enthält.

7. Dentalmaterial nach Anspruch 6, **dadurch gekennzeichnet, daß** die oberflächlich aufgebrachte Komponente im wesentlichen aus Polyethylen, Polyamid Polyester oder Glas besteht.

8. Dentalmaterial nach einem der Ansprüche 1 bis 7, **dadurch gekennzeichnet, daß** die polymerisierbare Komponente Füllstoff enthält.

9. Dentalmaterial nach Anspruch 8, **dadurch gekennzeichnet, daß** sie einen faserförmigen Füllstoff enthält.

10. Dentalmaterial nach einem der Ansprüche 1 bis 9 in vorgehärteter Form.

11. Verfahren zur Herstellung von Dentalrestaurationen, bei dem man auf ein Modell ein polymerisierbares Material aufbringt, man dann auf die Oberfläche des polymerisierbaren Materials eine Komponente aufbringt, die sich nach dem Härten des polymerisierbaren Materials unter Bildung einer rauhen Oberfläche entfernen läßt, man das polymerisierbare Material härtet und man dann die oberflächlich aufgebrachte Komponente unter Ausbildung einer rauhen Oberfläche von dem gehärteten polymerisierbaren Material entfernt.

12. Kit zur Herstellung von Dentalrestaurationen, enthaltend ein polymerisierbares Material und ein Abreißmaterial.

13. Kit nach Anspruch 12, **dadurch gekennzeichnet, daß** er als polymerisierbares Material einen Faserverbundwerkstoff enthält.

14. Kit nach Anspruch 12 oder 13, **dadurch gekennzeichnet, daß** das Abreißmaterial eine strukturierte Oberfläche aufweist.

15. Verwendung von Abreißmaterial zur Herstellung von Dentalmaterialien und/oder Dentalrestaurationen.
